# EUROPÄISCHE PATENTSCHRIFT

(11) **EP 1 049 416 B1**
(45) Veröffentlichungstag und Bekanntmachung des Hinweises auf die Patenterteilung: **03.01.2007**
(21) Anmeldenummer: 99924915.4
(22) Anmeldetag: 10.05.1999
(51) Int. Cl.: A61C 1/00, A61C 1/18, A61C 1/06, A61B 17/14, A61B 17/88

(54) **ARBEITSGERÄT FÜR BOHR-, SCHNEID- UND SCHRAUBWERKZEUGE ZU MEDIZINISCHEN ZWECKEN**
WORKING DEVICE FOR DRILLING, CUTTING AND SCREWDRIVER INSTRUMENTS USED FOR MEDICAL PURPOSES
DISPOSITIF D'ENTRAINEMENT POUR INSTRUMENTS DE PERçAGE, D'INCISION ET DE VISSAGE À USAGE MÉDICAL

(30) Priorität: 08.05.1998 DE 19820639; 08.05.1998 DE 19820640
(43) Veröffentlichungstag der Anmeldung: 08.11.2000
(73) Patentinhaber: Schmid, Heribert, 82194 Gröbenzell (DE)
(72) Erfinder: Schmid, Heribert, 82194 Gröbenzell (DE)
(74) Vertreter: TBK-Patent
(86) Internationale Anmeldenummer: PCT/EP1999/003174
(87) Internationale Veröffentlichungsnummer: WO 1999/058076

(56) Entgegenhaltungen:
- EP-A- 0 189 537
- EP-A- 0 812 578
- DE-A- 19 628 854
- DE-U- 29 712 012

## Beschreibung

Die vorliegende Erfindung bezieht sich auf ein elektrisches Antriebsgerät für Bohrer, Schneidwerkzeuge und Schraubbits, welche zu medizinischen Zwecken herkömmlicherweise verwendet werden sowie ein Verfahren zur Abstimmung des Antriebsgeräts und Überwachung des Zustands des Werkzeugs.

In der Medizin werden derartige Arbeitsgerätschaften beispielsweise in der Zahnmedizin für übliche Wurzelkanalaufbereitungen aber auch für das Aufbohren und Gewindeschneiden in Knochen sowie für das Eindrehen von Stiften verwendet. Auch werden diese Arbeiten manuell vom Zahnarzt bzw. vom Operateur durchgeführt, um Beschädigungen der zu behandelnden Zähne oder Knochen zu vermeiden.

Beispielsweise in der Zahnmedizin werden übliche Wurzelkanalaufbereitungen mittels eines elektrisch angetriebenen Bohrers ausgeführt, der in einem zahnmedizinischen Handstück gelagert ist. Für den Antrieb wird im allgemeinen ein kollektorloser Gleichstromantrieb-verwendet, wie er beispielsweise von der Firma KaVo unter der Bezeichnung "INTRAmatic LUX" vertrieben wird. Diese Art von Antrieb, welche normalerweise für den Betrieb von zahnmedizinischen Bohrwerkzeugen vorgesehen ist, wird mit dem in der Zahnmedizin eingesetzten Steuerungsaufwand in einem untersten Drehzahlbereich von circa 2000 bis 4000 Umdrehungen pro Minute bis in einen obersten Drehzahlbereich von circa 40000 bis 60000 Umdrehungen pro Minute bei einem Drehmoment von 2 bis 3 Ncm betrieben und sind in einem zahnmedizinischen Handstück eingebaut.

Zur Wurzelkanalaufbereitung werden indessen flexible Endo-Werkzeuge verwendet, mittels denen Wurzelkanäle, die unterschiedliche Durchmesser und unterschiedlichste Verläufe aufweisen können, ausgeräumt werden. Dies geschieht z. B. mit einem sich zur Spitze hin verjüngenden, flexiblen, spiralförmigen Bohrer von einer Länge von circa 20 bis 30 mm, der sich bei seiner Drehung in den Wurzelkanal schraubt, wobei durch Herausziehen dieses Bohrers der Kanal ausgeräumt wird. Laut Angabe der Hersteller dieser Art von Bohrern beträgt dessen Arbeitsdrehzahlbereich circa 100 bis 500 Umdrehungen pro Minute, in Sonderfällen bis 1800 Umdrehungen pro Minute. Um daher den für derartige Werkzeuge optimalen Arbeitsbereich zu erreichen, müssen bei den gegenwärtig vorgesehenen, für den Einbau in bekannte Griffstücke geeignete Elektromotoren mit dem vorstehend genannten Drehzahl-Spektrum von 2000 bis 40000 Umdrehungen pro Minute Untersetzungen von 1=16-20:1 nachgeschaltet werden. Durch derartige Getriebe wird zwar das Einsatzgebiet bekannter Elektromotoren für den medizinischen Bereich auch auf den Antrieb von flexiblen Spiralbohrern dieser Gattung erweitert. Jedoch bewirkt das Untersetzungsgetriebe gleichzeitig auch eine Drehmomenterhöhung um eben diesen Faktor, reduziert um den Wirkungsgradverlust des Getriebes.

Untersuchungen der Erfinderin haben ergeben, daß mit diesem Drehmoment nahezu alle zur Zeit eingesetzten Endo-Werkzeuge beispielsweise zur Wurzelkanalaufbereitung mehrfach über deren Bruchgrenze belastet werden. Es hat sich nämlich gezeigt, daß beispielsweise flexible Spiralbohrer für das Ausräumen von Wurzelkanälen bis nur maximal 0,2 Ncm belastet werden dürfen. Bei einem Überschreiten dieser Drehmomentgrenze kann der Spiralbohrer abbrechen und in dem zu behandelnden Wurzelkanal stecken bleiben. Derartige Unfälle lassen sich oft nur durch einen operativen Eingriff beheben.

Als ein relevanter Stand der Technik sei auf die DE 196 28 854 A verwiesen. Hieraus ist ein elektrisches Antriebsgerät für medizinische Bohr-, Schneid- und Schraubwerkzeuge bekannt mit jeweils einem vorbestimmten Umdrehungsbereich und einer maximal zulässigen Drehmomentbelastungsgrenze, wobei ein elektrischer Motor für den rotatorischen Antrieb des Werkzeugs vorgesehen ist.

Aus der EP-A-0 812 578 ist ebenfalls ein gattungsgemäßen elektrisches Antriebsgerät bekannt, das einen Schrittmotor als Antrieb für das Werkzeug verwendet. Ein solcher Schrittmotor hat dabei die Eigenschaft, dass er bei Erreichen einer maximalen Drehmomentgrenze außer Tritt fällt, d.h. dem vorgegebenen fixen Drehfeld nacheilt und schließlich stehen bleibt. Aus diesem Grund wird der aus diesem Stand der Technik bekannte Schrittmotor so ausgelegt, dass es im normalen Betrieb diese maximale Drehmomentgrenze nicht erreicht.

Angesichts dieses Stands der Technik liegt der Erfindung die Aufgabe zugrunde, ein elektrisches Antriebsgerät für medizinische Endo-Werkzeuge zu schaffen, dessen Drehzahl- und Drehmomentbereich unter geringem regelungstechnischen Aufwand derart variabel einstellbar ist, daß das elektrische Antriebsgerät bei nahezu sämtlichen Endo-Werkzeugen insbesondere für das Bohren und auch Gewinde Schneiden und Stift Eindrehen verwendet werden kann.

Diese Aufgabe wird erfindungsgemäß durch ein elektrisches Antriebsgerät für medizinische Werkzeuge mit den Merkmalen des Patentanspruchs 1 sowie durch ein erfindungsgemäßes Verfahren gemäß Anspruch 9 zur Anpassungen bzw. Eichung des Antriebsgeräts gelöst, wodurch ein exaktes Einstellen des maximalen Drehmoments ermöglicht wird.

Die Erfindung besteht demzufolge in der Ausbildung eines elektrischen Antriebsgeräts für medizinische Bohr-, Schneid- und Schraubwerkzeuge mit jeweils einem vorbestimmten Umdrehungsbereich und einer maximal zulässigen Drehmomentbelastungsgrenze, das einen elektrischen Schrittmotor umfaßt, dessen maximales Drehmoment und Drehzahl über die Stromstärke und die Drehfeldfrequenz vorgegeben werden kann. Umfangreiche Experimente erbrachten das Ergebnis, daß mit einem Schrittmotor als Antrieb für derzeit bekannte Endo-Werkzeuge deren Abbrechen im praktischen Einsatz verhindert wird. Hierbei macht man sich den Effekt des "außer Tritt Fallens" von Schrittmotoren zunutze, der maximal ein Blockieren des Motors bei Überlastung bewirkt. Der regelungstechnische Aufwand bleibt dabei äußerst gering und beschränkt sich im wesentlichen auf das Einstellen der Stromstärke und der Impulsgebung für den Schrittmotor.

Es ist ein besonderes Ziel der Erfindung, daß das vorstehend beschriebene Antriebsgerät für unterschiedliche Handstücke und somit unterschiedliche Triebwellenzüge verwendbar sein muß, die wiederum verschiedene Wirkungsgrade aufweisen. In anderen Worten ausgedrückt, stimmt die am Motor eingestellte maximale Drehmomentgrenze bei Handstücken nicht mit dem unmittelbar am Werkzeug maximal aufbringbaren Drehmoment überein. Das erfindungsgemäße Verfahren zur Abstimmung des Antriebsgeräts sieht demzufolge einen Selbsteichungsschritt (Kalibrierschritt) vor, der beispielsweise bei in Betriebnahme, nach einem Wechsel des Hand- oder Griffstücks bzw. des Triebwellenzugs, und/oder aber auch in bestimmten Zeitintervallen durchgeführt wird. Prinzipiell wird hierbei der erfindungsgemäße Schrittmotor mit einem vorbestimmten minimalen elektrischen Strom unterhalb des Losbrechstroms beaufschlagt. Daraufhin wird der Strom stufenweise erhöht, solange, bis der Schrittmotor anläuft. Der letzte Stromwert, der ein Anlaufen der Schrittmotors bewirkt, wird als jener Strom gespeichert, der zur Überwindung der Reibung des Triebwellenzugs notwendig ist und der folglich dem Strom des Antriebsgeräts proportional ist.

Das elektrisches Antriebsgerät gemäß der Erfindung ist dadurch weitergebildet, daß eine Abtriebswelle des Schrittmotors mit oder ohne Unter- oder Übersetzung an das Werkzeug gekoppelt ist, wobei der Schrittmotor vorteilhafterweise ohne Unter-/Übersetzung einen Arbeitsdrehzahlbereich von 100 bis 300 Umdrehungen pro Minute hat und bei einer Belastung gleich oder größer als sein eingestelltes Drehmoment außer Tritt fällt.

Ferner ist es vorgesehen, daß der Arbeitsdrehzahlbereich das Start-Stop-Geschwindigkeitsspektrum des Schrittmotors ist, innerhalb dem der Schrittmotor nach außer Tritt Fallen bei Verringerung der Drehmomentbelastung selbständig wieder anläuft, wobei es nach Anspruch 4 auch vorteilhaft ist, wenn der Schrittmotor auch außerhalb seines Start-Stop-Geschwindigkeitsspektrums bis zu einer Geschwindigkeit von 6000 Umdrehungen pro Minute betrieben werden kann.

Die vorstehend beschriebene Entwicklung ermöglicht somit den erweiterten Einsatz elektrischer Antriebe auf Werkzeuge mit geringer Bruchlast bei einer erheblichen Verringerung der Gefahr eines Abbrechens des Werkzeugs. Damit wird die Möglichkeit eröffnet, ein Werkzeug mehrmals zu verwenden. Es hat sich nunmehr herausgestellt, daß die von der Erfinderin zwischenzeitlich ermittelten Bruchbelastungsgrenzen für Endo-Werkzeuge mit zunehmender Einsatzdauer Veränderungen unterworfen sind. Im Rahmen von Versuchen konnte somit analytisch für einige Endo-Werkzeuge eine Maximal-Verwendungsdauer ermittelt werden, innerhalb der die Bruchwahrscheinlichkeit gering und die Schneidfähigkeit der Werkzeuge ausreichend ist. Bei Überschreiten dieser Maximal-Verwendungsdauer zeigten sich vermehrt Brüche in Folge von Materialermüdung sowie eine erhebliche Verschlechterung des erzielten Schnittbildes.

Um somit die Leistungsfähigkeit sowie das Sicherheitspotential des von der Erfinderin neu entwickelten Antriebskonzeptes voll ausnützen zu können, ist eine möglichst genaue Kenntnis über den Zustand des verwendeten Werkzeugs erforderlich, um in jedem Fall ein Abbrechen, nicht etwa in Folge eines unsachgemäßen Antriebs (Überlastung), sondern in Folge einer Materialermüdung bei zu langem Einsatz zu verhindern.

Angesichts dieser Problematik ist es nunmehr auch technisch sinnvoll, ein neues Verfahren zur Zustandserkennung eines medizinischen Werkzeugs, insbesondere eines Endo-Werkzeugs bereitzustellen.

Es wird ein Verfahren zur Überwachung des Zustands eines medizinischen Werkzeugs, insbesondere eines Endo-Werkzeugs vorgeschlagen, welches zumindest die nachfolgenden Schritte umfaßt:
a. Eingeben einer werkzeugspezifischen, d.h. bezüglich eines bestimmten Werkzeugs vorab ermittelten, maximal zulässigen Belastungsmenge in einen Rechner,
b) Erfassen einer Teilbelastungsmenge resultierend aus einer aktuellen Behandlung und Addieren dieser Teilbelastungsmenge zu einer zustandsspezifischen Gesamtbelastungsmenge resultierend aus vorhergehenden Behandlungen,
c. Vergleichen der maximal zulässigen Belastungsmenge mit der aktualisierten Gesamtbelastungsmenge und
d) Ausgeben eines Austauschsignals, falls die Gesamtbelastungsmenge die zulässige Belastungsmenge erreicht oder überschreitet.

Durch das vorstehend beschriebene Verfahren wird folglich für ein Werkzeug, z.B. einen flexiblen Spiralbohrer eine Maximalbelastungsmenge ermittelt für die das Risiko eines Bruchs minimal ist und fortlaufend die Ist-Belastungsmenge für dieses Werkzeug erfaßt und mit der Maximalbelastungsmenge verglichen. Auf diese Weise kann ein ausreichend guter Zustand des Werkzeugs gewährleistet werden.

Hierbei ist die Belastungsmenge als eine theoretische Größe definiert, welche aus der gefahrenen Umdrehungszahl bzw. Umdrehungsgeschwindigkeit, dem Drehmoment, sowie der Behandlungsdauer bestimmt wird. Zusätzlich oder alternativ kann die Anzahl an Sterilisationszyklen als Größe für die Belastungsmenge dienen.

Weitere vorteilhafte Ausführungen der Erfindung sind dabei Gegenstand der Unteransprüche.

Die Erfindung wird nachstehend anhand eines bevorzugten Ausführungsbeispiels unter Bezugnahme auf die begleitende Zeichnung näher erläutert.
Fig. 1 zeigt als ein mögliches Anwendungsbeispiel der Erfindung den generellen Aufbau eines in der Zahnmedizin allgemein verwendeten Winkelstücks zur Lagerung von Bohrern sowie zur Aufnahme eines elektrischen Antriebsmotors,
Fig. 2 zeigt ein Flußdiagramm bezüglich eines erfindungsgemäßen Arbeitsverfahrens bei einer Wurzelkanalbehandlung
Fig. 3 zeigt ein Flußdiagramm bezüglich eines Selbsteichungsprozesses.
Fig. 4 zeigt ein Flußdiagramm bezüglich eines Programmablaufs für eine Einsatzdauer-Überwachung und
Fig. 5 zeigt ein Flußdiagramm bezüglich des Programmablaufs für eine Sterilisationszyklus-Überwachung am Beispiel eines Spiralbohrers.

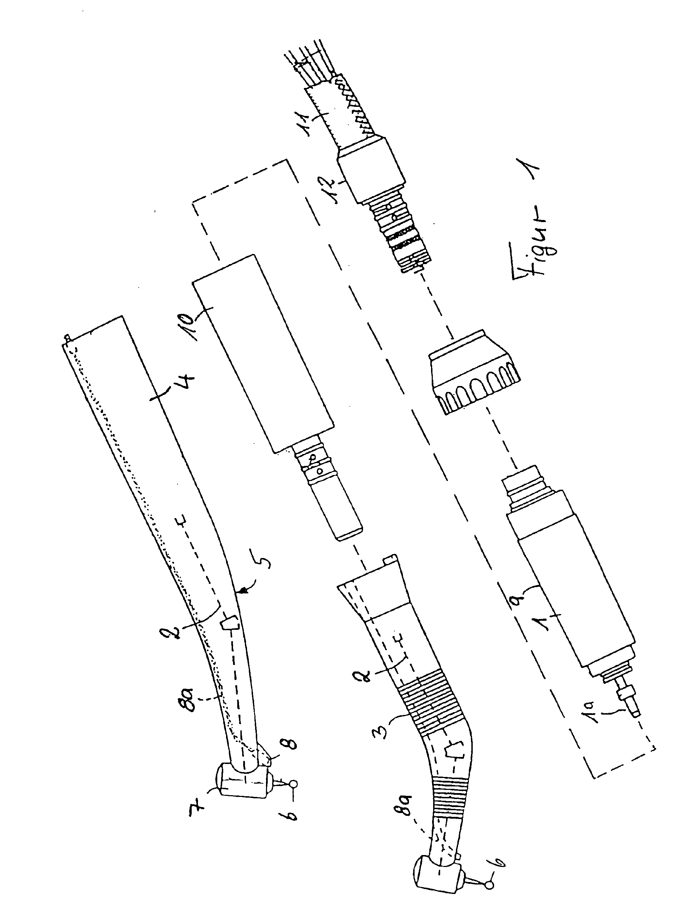

Wie aus der Fig. 1 zu entnehmen ist, besteht die zahnmedizinische Handstückanordnung aus einem an einem Versorgungsschlauchpaket 11 angeschlossenen elektrischen Antriebsmotor 1, an dessen freiem Wellenende 1a der Triebwellenabschnitt 2 eines eine gekrümmte Griffhülse 3 aufweisenden Handstückteils 5 angekoppelt ist. Das Handstückteil 5 selbst hat das Griffstück 3, an dessen äußerem Ende ein Bohrkopf 7 angeordnet ist, wobei an der gegenüberliegenden Stirnseite eine den Antriebsmotor 1 übergreifende weitere Hülse 4 befestigt ist. An dem Bohrkopf 7, welcher ein Werkzeug, vorliegend einen Dentalbohrer 6 drehbar aufnimmt, ist ferner eine Beleuchtungseinrichtung 8 mit einer Versorgungsleitung 8a angebracht.

Erfindungsgemäß ist der Antriebsmotor 1 als ein Schrittmotor ausgebildet, der die nachfolgenden Eigenschaften aufweist.

Der Schrittmotor 1 hat einen Arbeitsdrehzahlbereich von 0 bis 6000 Umdrehungen pro Minute und ca. 100 bis 300 Umdrehungen pro Minute innerhalb der sogenannten Start-Stop-Frequenz, wobei dessen Drehmomentbereich zwischen 0 und ca. 4 Ncm liegt. Der Schrittmotor 1 bestehend aus einem Rotor (nicht gezeigt) mit Abtriebswelle 1a und einem Stator (nicht gezeigt) umgeben von einem Außenmantel 9 bildet eine Motorpatrone, die in eine hülsenförmige Adapterpatrone 10 eingesteckt ist, das wiederum in dem Handstückteil, d. h. in der an der Griffhülse stirnseitig befestigten weiteren Hülse 4 axial eingeschoben und an der Griffhülse 3 befestigt ist. Der Anschluß zwischen dem Schrittmotor 1 und dem Versorgungsschlauchpaket 11 kann auf verschiedene Arten erfolgen.

Wie ferner in der Fig. 1 dargestellt wird, ist die Abtriebswelle 1a des Schrittmotors 1 unmittelbar an den Triebwellenabschnitt 2 für das Werkzeug 6 ohne ein Unter- oder Übersetzungsgetriebe angekoppelt. D. h., daß der Triebwellenabschnitt 2 mit der gleichen Umdrehungszahl dreht, wie die Abtriebswelle 1a des Schrittmotors 1. Natürlich kann in dem gekrümmten Griffstück 3 auch ein Untersetzungs- oder Übersetzungsgetriebe untergebracht sein, wobei das elektrische Antriebsgerät somit durch Austausch des jeweils verwendeten Griffstücks 3 wahlweise mit einem Untersetzungsgetriebe, mit einem Übersetzungsgetriebe oder ohne ein Getriebe lediglich durch Aufstecken des jeweiligen Griffstücks 3 auf die Adapterpatrone 10 ausgebildet werden kann.

Der Entwicklung des erfindungsgemäßen elektrischen Antriebsgeräts bestehend aus dem Schrittmotor 1 mit einer Umdrehungszahl von 0 bis 6000 Umdrehungen pro Minute und einem Drehmomentbereich zwischen 0 und 4 Ncm, der Abtriebswelle 1a des Schrittmotors 1 sowie dem Triebwellenabschnitt 2 sind zahlreiche Versuche insbesondere hinsichtlich der Belastungsfähigkeit der mit diesem elektrischen Antriebsgerät zu betreibenden Endo-Werkzeuge vorhergegangen, auf die nachfolgend kurz eingegangen werden soll:

Zuerst wurden Versuche mit flexiblen Endo-Werkzeugen für zahnmedizinische Zwecke insbesondere zur Wurzelkanalaufbereitung durchgeführt. Wie eingangs bereits kurz ausgeführt wurde, sind diese Endo-Werkzeuge in Form von flexiblen, spiralförmigen Bohrern ausgebildet, die sich zur Spitze hin verjüngen und vorzugsweise aus Nickel-TitanLegierung oder Stahl gefertigt sind. Die maximal zulässige Drehzahl derartiger Bohrer beträgt circa 100 bis 1800 Umdrehungen pro Minute. Die Versuche haben gezeigt, daß feine Bohrer dieser Gattung schon bei einem Drehmoment von circa 0,2 Ncm brechen, wobei bei Überschreiten dieser Drehmomentgrenze vorwiegend am äußersten Endbereich des Bohrers ein Bruch auftritt. Weitere praxisnahe Versuche mit derzeit bekannten elektrischen Antriebsgeräten zum Antreiben der vorstehend spezifizierten spiralförmigen Bohrer haben gezeigt, daß die herausgefundene maximale Drehmomentgrenze von 0,2 Ncm überschritten wird. Wurzelkanäle verlaufen in der Praxis nicht geradlinig sondern sind gekrümmt oder abgeknickt und weisen unterschiedliche Längen auf. Insofern tritt eine erhöhte Gefahr dahingehend auf, daß der Bohrer bei Erreichen einer derartigen Knickstelle innerhalb des Wurzelkanals oder bei Erreichen des Wurzelkanalendes bricht, da das elektrische Antriebsgerät ein zu hohes Abtriebsmoment aufweist. Dies geschieht so schnell und überraschend, daß der behandelnde Arzt keine Möglichkeiten hat, dem Abbrechen des Bohrers beispielsweise durch rechtzeitiges Ausschalten oder Rückziehen des elektrischen Antriebsgerätes vorzubeugen.

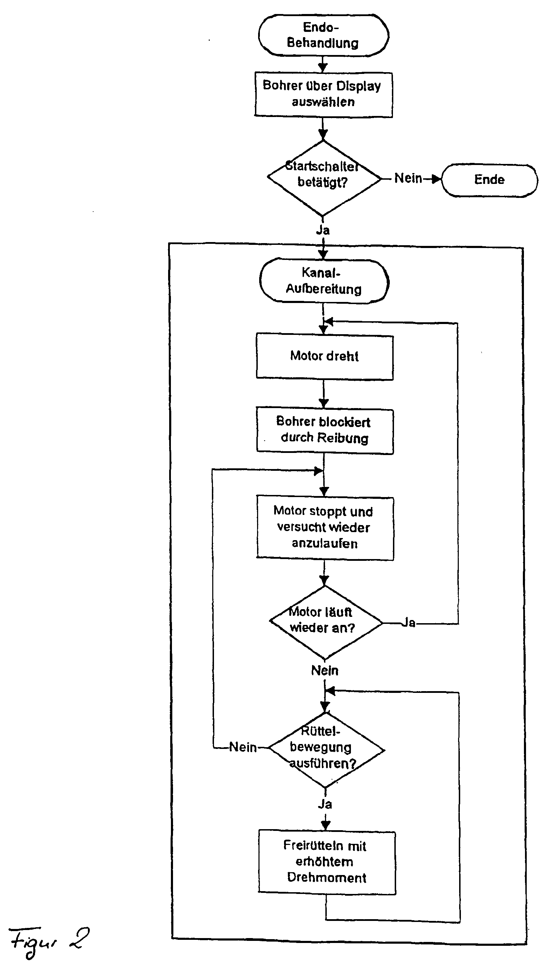

Das erfindungsgemäße elektrische Antriebsgerät verwendet indessen gemäß vorstehender Beschreibung den Schrittmotor 1 und macht sich dabei bei einem Arbeitsvorgang gemäß Fig. 2 beispielsweise für eine Wurzelkanalbehandlung die folgenden Eigenschaften des Schrittmotors zunutze:

Der verwendete Schrittmotor 1 läßt sich konstruktiv bedingt bereits in einem Drehzahlbereich zwischen 100 und 300 Umdrehungen pro Minute innerhalb seiner Start-Stop-Frequenz betreiben. D. h. wird der Motor über dessen eingestelltes maximales Drehmoment belastet, fällt er außer Tritt und versucht selbständig wieder anzulaufen und läuft auch an, wenn die Drehmomentanforderung wieder zurückgeht. Dieses "außer Tritt Fallen" ist nicht nur hörbar sondern auch durch Vibrationen an der Handstückanordnung für den behandelnden Arzt eindeutig spürbar. Das "außer Tritt Fallen" des Schrittmotors verhindert im Zeitraum zwischen dem Auftreten der Überbelastung des Schrittmotors und dem Erkennen bzw. Reagieren durch den Arzt eine Überlastung des Werkzeugs, nämlich des Spiralbohrers über die eingestellte maximale Drehmomentgrenze hinaus, so daß in jedem Fall ein Bruch infolge einer Drehmomentüberlastung verhindert wird.

Des weiteren ermöglicht der Direktantrieb des Werkzeuges durch das elektrische Antriebsgerät ohne Untersetzungs- oder Übersetzungsgetriebe eine im wesentlichen genaue Einstellung des maximal erreichbaren Drehmoments an dem Werkzeug durch Bestimmung der maximal zulässigen Stromstärke sowie der Drehzahl über die Drehfeldfrequenz des Schrittmotors. Es lassen sich bei dem erfindungsgemäßen Schrittmotor jedoch nicht nur Drehzahl und Drehmoment exakt einstellen sonder auch dessen Drehrichtung, wodurch die folgende Funktion ermöglicht wird.

Unter der Annahme, daß sich beispielsweise der Bohrer zum Ausräumen eines Wurzelkanals festgefressen und das Antriebsgerät in Folge der überhöhten Drehmomentbelastung außer Tritt gefallen ist, so ist der Schrittmotor innerhalb der Start-Stop-Frequenz bestrebt, wieder anzulaufen. Dies führt zu einer Vibration oder Rüttelbewegung, die der Arzt am Griffstück fühlt. Es besteht nunmehr die Möglichkeit den Schrittmotor bzw. dessen Drehfeldfrequenz wie in der Fig. 2 dargestellt ist, derart zu steuern, daß der Motor abwechselnd die Drehrichtung ändert also eine gezielte, definierte Hin- und Herbewegung (beispielsweise ein sogenannter Pilgerschritt, bei dem eine Vorwärtsdrehung jeweils größer ist als die darauffolgende Rückwärtsdrehung) ggf. mit einer erhöhten Stromstärke, die proportional dem Drehmoment ist, das unterhalb des maximal zulässigen Drehmoments liegt und diese solange ausführt, bis sich der Bohrer wieder losgerissen hat, um dann in die Hauptdrehrichtung weiterzudrehen. Dieser Verstärkermodus kann entweder manuell durch den Arzt oder automatisch bei Überschreiten des maximalen Drehmoments eingeleitet werden.

Des weiteren hat sich gezeigt, daß sich auch solche Werkzeuge mittels des erfindungsgemäßen elektrischen Antriebsgerätes betreiben lassen, die bei einer höheren Drehzahl z. B. bei 2000 Umdrehungen pro Minute arbeiten, also außerhalb der Start-Stop-Drehzahl. Der erfindungsgemäße Schrittmotor kann in diesem Fall über eine definierte Beschleunigungsfunktion auf diese Geschwindigkeit beschleunigt werden und fällt dabei ebenfalls außer Tritt, d. h. er bleibt in diesem Falle stehen, wenn das den Schrittmotor beaufschlagende Drehmoment dessen eingestellte maximale Drehmomentgrenze überschreitet. Wird der erfindungsgemäße Schrittmotor in diesem Drehzahlbereich von circa 2000 Umdrehungen pro Minute betrieben, kann jedoch der Schrittmotor, wenn er einmal außer Tritt gefallen ist, nicht mehr selbständig anlaufen. Um den Elektromotor daher wieder auf diese Drehzahl zu beschleunigen, muß die Drehfeldfrequenz manuell beispielsweise über einen Fußschalter unter die maximale Start-Stop-Geschwindigkeit abgesenkt werden, damit der Motor von neuem auf die durch das verwendete Werkzeug vorgeschriebene Arbeitsgeschwindigkeit hochgefahren werden kann.

Aus der vorstehenden Beschreibung des erfindungsgemäßen elektrischen Antriebsgerätes läßt sich erkennen, daß dieses zu zahlreichen anderen medizinischen Zwecken angewendet werden kann. Da die Drehzahl sowie das Drehmoment leicht und exakt dem Schrittmotor vorgegeben werden können und damit auch die Drehzahl sowie das maximale Drehmoment an dem Werkzeug eingestellt werden kann, lassen sich unter anderem auch Gewinde in vorgebohrten Bohrungen schneiden, wie sie beispielsweise in der Knochenchirurgie benötigt werden. Das Gewinde reißt nicht aus, da bei Überlastung des Motors entsprechend dessen maximaler Drehmomentgrenze der Motor einfach stehen bleibt und nur versucht, wieder anzulaufen. Auch läßt sich bei dem verwendeten Schrittmotor die Drehrichtung in einfacher Weise umkehren, so daß Bohrer oder Gewindebohrer bei Erreichen eines Punktes bzw. Eindringtiefe in exakter Weise wieder herausgefahren werden kann. Das gleiche gilt natürlich auch für das Eindrehen von Gewindestiften in diese Gewindebohrungen.

Als eine zusätzliche Einrichtung für das erfindungsgemäße Arbeitsgerät kann ein elektronischer Schaltpult vorgesehen sein, in dem eine Vielzahl von unterschiedlichen Werkzeugtypen sowie deren technische Daten abgespeichert sind. Auf diese Weise braucht der behandelnde Arzt lediglich den von ihm benutzten Werkzeugtyp einzugeben oder aufzurufen, um das Arbeitsgerät leistungstechnisch auf die zulässigen Werte dieses Werkzeugs einzustellen.

Als weitere mögliche Anwendungsbereiche seien die Folgenden genannt:
1. Im Falle einer Kariesbehandlung läßt sich der Umstand zunutze machen, daß ein Karies befallener Zahnschmelz eine geringere Härte besitzt, als ein gesunder Zahnschmelz. D.h. erkrankte Zahnbereiche verändern ihre Struktur und damit ihre Festigkeit und Härte gegenüber gesunden Zahnbereichen. Der Schrittmotor kann nunmehr so eingestellt werden, daß das maximal abgebbare Drehmoment zwar ausreicht, einen Bohrer in das erkrankte Zahnmaterial zu treiben, um dieses auszuräumen. Sobald jedoch der Bohrer auf den gesunden Zahnschmelz trifft, überschreitet das aufgebrachte Drehmoment diese maximale Drehmomentgrenze, worauf der Schrittmotor stoppt. Gesunder Zahnschmelz bleibt dadurch nahezu vollständig erhalten. Im übrigen verursacht ein derart betriebener Bohrer geringere Vibrationen, die im Falle des Entfernes von gesundem Zahnschmelz verstärkt auftreten würden, wodurch Schmerzen, verursacht durch eine Traumatisierung, d.h. ein Zusammensrücken der Nervenkammer während der Zahnbehandlung, reduziert werden.
2. Gleiches gilt auch beispielsweise bei der Entfernung von Zahnstein, welches gegenüber dem Zahnschmelz ebenfalls andere Eigenschaften bezüglich Härte und Festigkeit besitzt, auf die das erfindungsgemäße Antriebsgerät hinsichtlich einer optimalen Drehzahl und Drehmomentgrenze eingestellt werden kann.

Wie eingangs kurz erwähnt wurde, soll das erfindungsgemäße Antriebsgerät sowohl hinsichtlich seines Verwendungsbereichs (unterschiedliche medizinische Einsatzmöglichkeiten) als auch hinsichtlich unterschiedlicher Handstückkonstruktionen möglichst flexibel sein. Im Rahmen der zahlreichen Versuche wurde herausgefunden, daß trotz exakt einstellbarer Drehzahlen und Drehmomente am Schrittmotor selbst, Abweichungen bzw. Drehzahl- und/oder Drehmomentschwanken direkt am Werkzeug für unterschiedliche Handstücke auftreten. Der Grund hierfür besteht darin, daß insbesondere Winkelstücke mit unterschiedlichen Abkrümmwinkeln, Materialien, Antriebswellenzügen, Lagerungen usw., kurz gesagt, Handstücke unterschiedlicher Hersteller voneinander abweichende Wirkungsgrade besitzen. D.h. wird ein bestimmtes Drehmoment am erfindungsgemäßen Schrittmotor eingestellt, so wird das tatsächlich am Bohrer erreichbare maximale Drehmoment entsprechend dem Wirkungsgrad (Drehmomentverlust z.B. durch Reibung usw.) entsprechend des verwendeten Handstücks verringert. Die Folge hiervon ist, daß eine exakte Einstellung des Drehmoments am Werkzeug praktisch unmöglich wird.

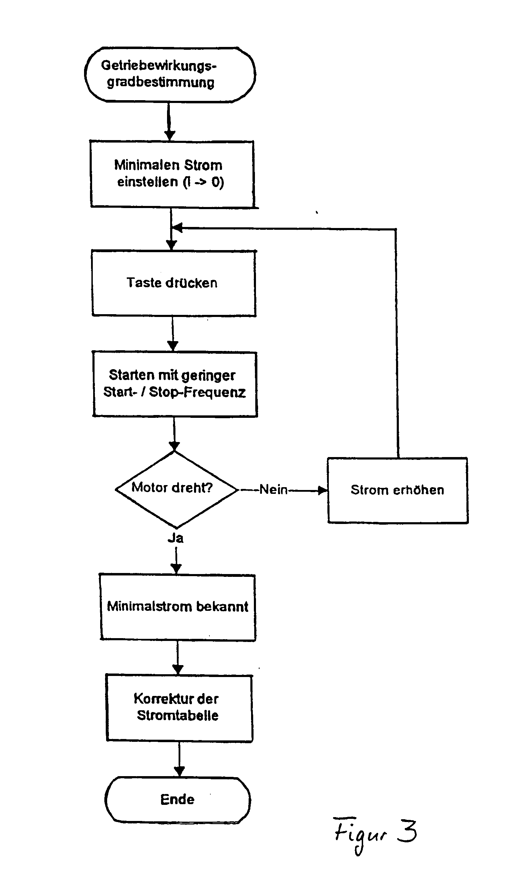

Die Lösung dieses Problem besteht in dem erfindungsgemäßen Selbsteinchungsprozeß, wie er in der Fig. 3 dargestellt ist.

Gemäß dieser Fig. 3 wird für eine Getriebe- bzw. Triebwellenzug-Bestimmung z.B. nach einem Wechsel des Griffstücks, bei jeder Inbetriebnahme des Antriebsgeräts und/oder aber auch grundsätzlich in vorbestimmten Zeitintervallen durch den Arzt ein Selbsteichungsprozeß in Form eines bestimmten Leerlaufprogramms eingeleitet. Dieses Programm wird gestartet durch Betätigen einer nicht weiter aufgeführten Eichungs-Starttaste, worauf der Schrittmotor in einem ersten Verfahrensschritt mit einem minimalen elektrischen Strom beaufschlagt wird. Dieser Minimalstrom wird unterhalb des Schrittmotor eigenen Losbrechstroms gewählt oder ist bereits standartgemäß auf diesen Wert voreingestellt, so daß der Motor bei Anlegen dieses Stroms nicht anläuft.

Nach jeweils einer bestimmten Anzahl von Drehfeldfortschaltungen wird der elektrische Strom um ein vorbestimmtes Maß stufenweise erhöht, solange, bis der Schrittmotor anläuft. Dieses Anfahren des Schrittmotors wird entweder visuell/akustisch durch den Arzt oder über einen Sensor erfaßt, worauf die in Fig. 3 dargestellte Stromerhöhungs-Programmschleife des Eichungsprozesses manuell oder automatisch beendet wird. Mit Beendigung dieser Programmschleife, schreitet der Vorgang zum nächsten Schritt fort, in welchem der zuletzt eingestellte elektrische Strom als Losbrechstrom in einer Tabelle abgespeichert wird. Dieser Losbrechstrom stellt gleichzeitig auch den Verluststrom durch das verwendete Winkelstück und damit indirekt einen Wert für dessen Wirkungsgrad dar.

Wird nunmehr die gewünschte Stromstärke entsprechend dem zu verwendenden Werkzeug oder der beabsichtigten Behandlungsart vom Arzt eingegeben, addiert ein das Programm ausführender Rechner automatisch den zuvor gemessenen Verluststrom zu dem eingegebenen Wert hinzu, um den zu erwartenden Verlust durch das Winkelstück oder ein nachfolgendes Getriebe auszugleichen. Auf diese Weise entspricht das maximale Drehmoment am Werkzeug sehr exakt dem eingegebenen Stromwert.

Die vorstehend beschriebene Eichung kann alternativ auch bereits durch die Hersteller der Winkelstücke selbst standartgemäß durchgeführt und in Form eines elektrisch lesbaren oder auch manuell in den Rechner eingebbaren Codes beispielsweise auf dem Gehäuse des Winkelstücks angegeben werden. D.h. Winkelstücke, Handstücke, Triebwellenzüge usw. werden ab Werk mit einer Wirkungsgradangabe versehen, die dann entweder der Arzt bei der Eingabe des Maximalstromwerts berücksichtigt oder aber vom Rechner über einen geeigneten Sensor erkennt und als Verluststrom bei der Korrektur der manuell eingegebenen Stromtabelle verwendet.

Die Erfindung bezieht sich demzufolge auf ein elektrisches Antriebsgerät für medizinische Anwendungen insbesondere zum Antrieb von Bohrern, Schneid- und Schraubwerkzeugen. Derartige Endo-Werkzeuge haben vorbestimmte Arbeitsdrehzahlbereiche und besitzen entsprechend deren Materialien und Dimensionen maximal zulässige Drehmomentbelastungsgrenzen. Um ein Überschreiten dieser individuellen Drehmomentbelastungsgrenzen zu vermeiden, ist das Antriebsgerät mit einem Schrittmotor ausgerüstet. Der Schrittmotor fällt bei Erreichen dessen maximaler Drehmomentgrenze außer Tritt, wobei die maximale Drehmomentgrenze des Schrittmotors unterhalb der Drehmomentsbelastungsgrenze des Werkzeuges festgelegt wird. Um die Drehmomentbelasungsgrenze exakt einstellen zu können, führt das Antriebsgerät einen Eichungsvorgang im Rahmen eines Leerlaufs durch, bei dem der Wirkungsgrad des verwendeten Triebwellenzugs bis zum Werkzeug erfaßt und damit der Stromwert für ein Null-Drehmoment ermittelt wird.

Für die nachfolgende Beschreibung des Zustandsüberwachungsverfahrens gemäß einem bevorzugten Ausführungsbeispiel der Erfindung sei darauf hingewiesen, daß eine Art Magazin für unterschiedliche Werkzeuge in Form einer Anzahl von Behältern oder Kästchen vorbereitet ist, in denen die Werkzeuge nummeriert oder anderweitig codiert gebunkert sind. Ein nicht weiter dargestellter Rechner, welchem die Anzahl, Lage, und/oder Art der einzelnen Werkzeuge vorab eingegeben worden ist, verwaltet diese Behälter und ordnet jedem Werkzeug zustandsspezifische Werte zu, wie nachfolgend noch beschrieben wird.

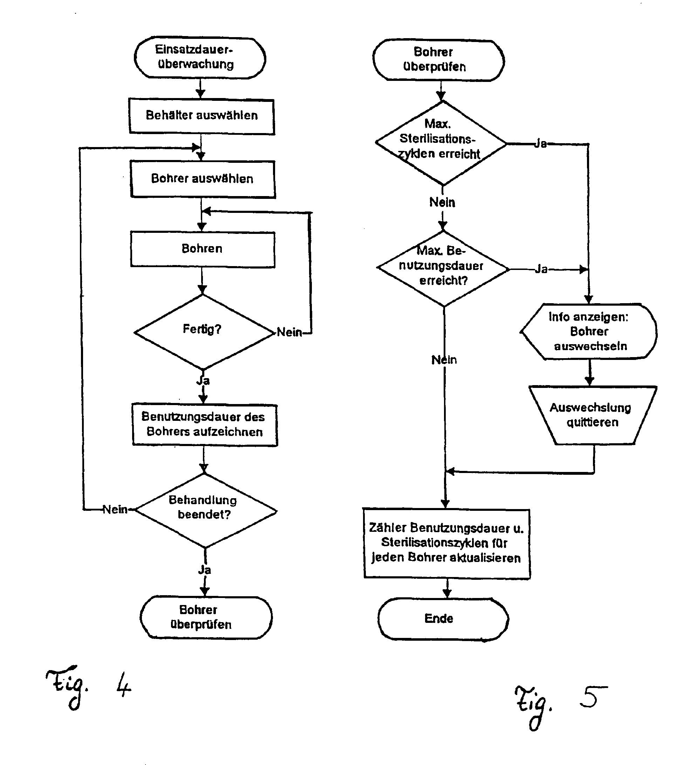

Das Einsatzdauer-Überwachungsprogramm gemäß der Fig. 4 kann entweder manuell vor Beginn einer Behandlung oder automatisch bei jeder Inbetriebnahme eines elektrischen Antriebsgeräts insbesondere eines Schrittmotors gestartet werden, der im Fall einer Wurzelbehandlung eingesetzt wird.

Zu Beginn wählt der behandelnde Arzt aus dem Magazin einen Behälter, vorzugsweise gefüllt mit nur der Art von Werkzeugen, welche für die beabsichtigte Behandlung besonders geeignet ist, aus und entnimmt eines dieser Werkzeuge aus dem Behälter. Anschließend gibt der Arzt den Code dieses Werkzeugs in den Rechner ein. Alternativ hierzu kann der Rechner mit einer geeigneten Sensorik ausgestattet sein, die ihm ein automatisches Einlesen und Erkennen des ausgewählten Werkzeugs ermöglicht.

Wie eingangs bereits angedeutet wurde, lassen sich für jedes beispielsweise in der Zahnmedizin verwendete Werkzeug spezielle Werte hinsichtlich max. Umdrehungszahl und Belastung, Einsatzdauer usw. sowie eine Maximalzahl an Sterilisationszyklen analytisch ermitteln, aus denen eine maximale Belastungsmenge als eine theoretische Größe bestimmt werden kann, bei deren Überschreiten ein erheblicher Anstieg an Ermüdungsbrüchen festzustellen ist. Diese maximal zulässigen Werte bzw. die max. Belastungsmenge werden dem Rechner für jedes im Magazin befindliche Werkzeug bei dessen Einsortieren entweder manuell oder automatisch per Sensor eingegeben, so daß der Rechner bei entsprechendem Code-Aufruf die werkzeugspezifischen Werte aufrufen kann.

Nachdem das Werkzeug, beispielsweise ein flexibler Spiralbohrer für eine Wurzelkanalaufbereitung, in das Handstück eingesetzt und der Rechner über das verwendete Werkzeug informiert ist, beginnt der Arzt mit der Behandlung, in dem das Antriebsgerät angefahren wird. Mit Anfahren des Antriebsgeräts mißt der Rechner fortlaufend beispielsweise die Betriebszeitdauer, die Stromhöhe als Ausgangswert zur Bestimmung der Belastungshöhe, die Drehzahl und/oder im Falle eines Schrittmotors die Anzahl von Takten als Referenzwert gefahrener Umdrehungen und speichert diese Werte als zustandsspezifische Werte in einem Zwischenspeicher ab.

Nach Beendigung der Behandlung schaltet der Arzt das Antriebsgerät ab oder gibt alternativ ein Beendigungssignal dem Rechner ein, um diesem das Behandlungsende für dieses Werkzeug anzuzeigen. Der Rechner wertet nunmehr die gemessenen und abgespeicherten zustandsspezifischen Werte aus, welche ein Belastungsprofil für die vergangene Behandlung darstellen und errechnet hieraus eine theoretische Teilbelastungsmenge. Diese Teilbelastungsmenge wird nunmehr einer Gesamtbelastungsmenge aus ggf. vorhergehenden Behandlungen mit dem selben Werkzeug hinzu addiert, um somit die Gesamtbelastungsmenge für dieses Werkzeug zu aktualisieren. Hierauf wird dieses Programm beendet.

Nach Beendigung des vorstehend beschriebenen Programmablauf führt der Rechner ein Sterilisationszyklen-Überwachungsprogramm aus.

Das vom Arzt verwendete Werkzeug wird nach der Behandlung in den Behälter zurückgesteckt. Behälter und Werkzeuge werden anschließend gereinigt und einem Sterilisationsvorgang unterzogen. Es hat sich in Versuchen gezeigt, daß dieser Sterilisationsvorgang die Werkzeuge angreift und zu einer beschleunigten Materialalterung insbesondere einem Abstumpfen der Werkzeuge führt. Aus diesem Grunde zählt der Rechner nach jeder Behandlung die Anzahl an Sterilisationszyklen, um somit die Gesamtzahl an Sterilisationszyklen für dieses verwendete Werkzeug zu aktualisieren.

Der Rechner vergleicht nunmehr gemäß der Fig. 5 nach Aktualisieren der tatsächlichen zustandsspezifischen Werte, insbesondere der Gesamtzahl an Sterilisationszyklen sowie der Gesamtbelastungsmenge diese Ist-Werte mit den maximal zulässigen, werkzeugspezifischen Werten für das verwendete Werkzeug und gibt bei Erreichen oder Überschreiten eines der Maximalwerte ein Warnsignal aus, welches dem Arzt den notwendigen Austausch dieses Werkzeugs anzeigt. In diesem Fall wird der Arzt das Werkzeug nicht mehr im Behälter ablegen, sondern durch ein Neues ersetzen.

Wie aus der vorstehenden Beschreibung ersichtlich ist, wird in diesem Ausführungsbeispiel erst nach Beendigung einer Behandlung der Ist-Zustand des Werkzeugs ermittelt und mit dem maximal zulässigen Zustand verglichen. Es kann jedoch der Fall auftreten, daß bereits während einer Behandlung beispielsweise die maximale Benutzungsdauer d.h. die vorbestimmte maximale Gesamtbelastungsmenge erreicht oder sogar überschritten wird. Um dieses Problem auf einfache Weise zu lösen, wird gemäß dem bevorzugten Ausführungsbeispiel bei der Bestimmung der maximalen Gesamtbelastungsmenge ein Sicherheitsfaktor mit eingerechnet, der derart gewählt ist, daß im Rahmen einer durchschnittlichen Behandlung mit dem entsprechenden Werkzeug diese tatsächliche max. Belastungsmenge nicht erreicht werden kann.

Alternativ zu dieser einfachen Vorgehensweise, könnte insbesondere der Programmablauf für die Einsatzdauer-Überwachung gemäß der Fig. 4 derart geändert werden, daß eine zusätzliche Programmschlaufe "Erfassen des Werkzeugzustands und Vergleich des Ist-Werkzeugzustand mit der max. Gesamtbelastungsmenge" vor dem in Fig. 4 gezeigten Schritt "Bohrer prüfen" eingefügt wird, so daß ein entsprechendes Warnsignal ggf. auch während der Behandlung an den Arzt ausgegeben werden kann. In diesem Fall könnte der Sicherheitsfaktor für jedes Werkzeug reduziert und die Standzeit des Werkzeugs noch weiter verlängert werden.

## Patentansprüche

1. Elektrisches Antriebsgerät für medizinische Bohr-, Schneid- und Schraubwerkzeuge (6) mit jeweils einem vorbestimmten Umdrehungsbereich und einer maximal zulässigen Drehmomentbelastungsgrenze, **gekennzeichnet durch** einen elektrischen Schrittmotor (1), dessen maximales Drehmoment und dessen Drehzahl über die Stromstärke und die Drehfeldfrequenz vorgebbar sind, derart, dass der Schrittmotor innerhalb seines über die Drehfeldfrequenz eingestellten Arbeitsdrehzahlbereichs bei Erreichen seiner über die Stromstärke eingestellten maximalen Drehmomentgrenze außer Tritt fällt, wobei die maximale Drehmomentgrenze unterhalb der Drehmomentbelastungsgrenze des Werkzeugs festgelegt wird und wobei der Arbeitsdrehzahlbereich das Start-Stop Geschwindigkeitsspektrum des Schrittmotors (1) ist, innerhalb dem der Schrittmotor (1) nach außen Tritt Fallen bei Verringerung der Drehmomentbelastung selbstständig wieder anläuft.

2. Elektrisches Antriebsgerät nach Anspruch 1, **dadurch gekennzeichnet, dass** eine Abtriebswelle (1a) des Schrittmotors (1) mit/ohne Unter- oder Übersetzung an das Werkzeug (6) gekoppelt ist.

3. Elektrisches Antriebsgerät nach einem der vorstehenden Ansprüche, **dadurch gekennzeichnet, dass** der Schrittmotor (1) ohne Unter-/Übersetzung einen Arbeitsdrehzahlbereich von 100 bis 300 Umdrehungen pro Minute hat und bei einer Belastung gleich oder größer als sein eingestelltes Drehmoment außer Tritt fällt.

4. Elektrisches Antriebsgerät nach einem der vorstehenden Ansprüche, **dadurch gekennzeichnet, dass** der Schrittmotor (1) außerhalb seines Start-Stop-Geschwindigkeitsspektrums bis zu einer Geschwindigkeit von 6000 Umdrehungen pro Minute betreibbar ist.

5. Elektrisches Antriebsgerät nach einem der vorstehenden Ansprüche, **dadurch gekennzeichnet, dass** das Abgabedrehmoment des Schrittmotors (1) zwischen 0 und 4Ncm ist.

6. Elektrisches Antriebsgerät nach einem der vorstehenden Ansprüche, **dadurch gekennzeichnet, dass** der Schrittmotor (1) zumindest bei Erreichen oder Überschreiten des maximalen Drehmoments in einem Verstärkermodus regelbar ist, in dem der Schrittmotor eines definierte Links-Rechts-Bewegung beispielweise einen Pilgerschritt ausführt, um dann bei Unterschreiten des maximalen Drehmoments in seine Vorzugsrichtung weiter zu drehen.

7. Elektrisches Antriebsgerät nach Anspruch 6, **dadurch gekennzeichnet, daß** die Links-Rechts-Bewegung jeweils auf eine vorbestimmte Gradzahl beschränkt ist.

8. Elektrisches Antriebsgerät nach einem der vorstehenden Ansprüche **dadurch gekennzeichnet, daß** das Antriebsgerät ein zahnmedizinisches Handstück mit einem Winkelstück ist, in dem ein Triebwellenzug lagert, der durch den Schrittmotor (1) antreibbar ist.

9. Verfahren zur Selbsteichung eines elektrischen Antriebsgeräts mit den Merkmalen gemäß einem der vorstehenden Ansprüche, **gekennzeichnet durch** folgende Verfahrensschritte:
a. Beaufschlagen des Motors (1) mit einem Minimalstrom unterhalb des motorspezifischen Losbrechstroms,
b. schrittweises Erhöhen des Stroms nach bestimmten Drehfeldfortschaltungen,
c. Erfassen des Stromwerts, bei welchem der Motor (1) anläuft.

10. Verfahren nach Anspruch 9 **dadurch gekennzeichnet, daß** der erfaßte Stromwert als Verluststrom abgespeichert und dem eingegebenen Maximalstrom hinzu addiert wird.

11. Verfahren nach einem der Ansprüche 9 und 10, **dadurch gekennzeichnet, daß** die Selbsteichung bei Inbetriebnahme des Motors (1) und/oder bei einem Wechsel des Antriebsgeräts und/oder nach einer vorbestimmten oder bestimmbaren Zeitperiode ausgeführt wird.

12. Verfahren nach einem der vorstehenden Ansprüche **dadurch gekennzeichnet, daß** der Verluststrom in Form eines vorzugsweise am Antriebsgerät bereits angebrachten, elektronisch erfaßbaren Codes oder durch den Arzt manuell eingegeben wird, wobei der Verluststrom werksseitig bereits ermittelt wird.

## Claims

1. Electrical drive device for medical boring, cutting and screwing instruments (6) having respectively a predetermined speed range and a maximum permissible torque loading limit, **characterised by** an electrical step motor (1), the maximum torque and the speed of which are prescribable via the current strength and the rotational field frequency, such that the step motor slips out of synchronism upon reaching its maximum torque limit which is set via the current strength, within its operating speed range which is set via the rotational field frequency, the maximum torque limit being specified below the torque loading limit of the tool, and the operating speed range being the start-stop speed spectrum of the step motor (1), within which the step motor (1) automatically starts again after slipping out of synchronism upon reduction of the torque loading.

2. Electrical drive device according to claim 1, **characterised in that** a driven shaft (1a) of the step motor (1) is connected to the instrument (6) with/without stepping down or up.

3. Electrical drive device according to one of the preceding claims, **characterised in that** the step motor (1), without stepping down/up, has an operating speed range of 100 to 300 revolutions per minute and slips out of synchronism at a loading equal to or greater than its set torque.

4. Electrical drive device according to one of the preceding claims, **characterised in that** the step motor (1) can be operated outwith its start-stop speed spectrum up to a speed of 6000 revolutions per minute.

5. Electrical drive device according to one of the preceding claims, **characterised in that** the output torque of the step motor (1) is between 0 and 4 Ncm.

6. Electrical drive device according to one of the preceding claims, **characterised in that** the step motor (1) can be controlled, at least upon reaching or exceeding the maximum torque, in a booster mode, in which the step motor implements a defined left-right movement, for example a pilger step, in order then, upon falling below the maximum torque, to rotate further in its preferred direction.

7. Electrical drive device according to claim 6, **characterised in that** the left-right movement is restricted respectively to a predetermined number of degrees.

8. Electrical drive device according to one of the preceding claims, **characterised in that** the drive device is a dental handpiece with an elbow, in which a drive shaft cable, which can be driven by the step motor (1), is mounted.

9. Method for self-calibration of an electrical drive device with the features according to one of the preceding claims, **characterised by** the following method steps:
a) supplying the motor (1) with a minimum current below the motor-specific breakaway current,
b) increasing the current stepwise according to specific rotational field rapid reclosing,
c) detecting the current value at which the motor (1) starts.

10. Method according to claim 9, **characterised in that** the detected current value is stored as leakage current and is added on to the input maximum current.

11. Method according to one of the claims 9 and 10, **characterised in that** the self-calibration is implemented when starting the motor (1) and/or when changing the drive device and/or after a predetermined or determinable period of time.

12. Method according to one of the preceding claims, **characterised in that** the leakage current is input in the form of a code which is preferably already provided on the drive device and can be detected electronically or is input manually by the dentist, the leakage current being determined already in the factory.

## Revendications

1. Dispositif électrique d'entraînement pour des instruments (6) de perçage, d'incision et de vissage à usage médical avec pour chacun une plage de rotation prédéterminée et une limite de charge de couple de rotation maximale fiable, **caractérisé par** un moteur pas à pas (1) électrique, dont le couple de rotation maximal et le régime sont prédéterminés par l'intensité du courant et par la fréquence du champ tournant, de manière telle que le moteur pas à pas fonctionne hors synchronisme à l'intérieur de sa plage de régime de travail réglé par la fréquence de champ tournant lorsqu'il atteint sa limite de couple de rotation maximale réglée par l'intensité du courant, la limite de couple de rotation maximale étant définie en dessous de la limite de charge de couple de rotation de l'instrument, et la plage de régime de travail étant le spectre de vitesse marche/arrêt du moteur pas à pas, à l'intérieur duquel le moteur pas à pas (1) redémarre de manière autonome après qu'il a fonctionné hors synchronisme lors de la diminution de la charge de couple de rotation.

2. Dispositif électrique d'entraînement selon la revendication 1, **caractérisé en ce qu'**un arbre d'entraînement (1) est couplé à l'instrument (6) avec ou sans multiplication ou démultiplication.

3. Dispositif électrique d'entraînement selon l'une quelconque des revendications précédentes, **caractérisé en ce que** le moteur pas à pas (1) sans démultiplication / multiplication présente une plage de régime de travail de 100 à 300 tours par minute et fonctionne hors synchronisme lorsqu'une charge est supérieure ou égale à son couple de rotation réglé.

4. Dispositif électrique d'entraînement selon l'une quelconque des revendications précédentes, **caractérisé en ce que** le moteur pas à pas (1) peut fonctionner en dehors de son spectre de vitesse marche/arrêt jusqu'à une vitesse de 6000 tours par minute.

5. Dispositif électrique d'entraînement selon l'une quelconque des revendications précédentes, **caractérisé en ce que** le couple de rotation de sortie du moteur pas à pas (1) est entre 0 et 4 Ncm.

6. Dispositif électrique selon l'une quelconque des revendications précédentes, **caractérisé en ce que** le moteur pas à pas (1) peut être réglé au moins lorsque le couple de rotation maximal est atteint ou dépassé dans un mode d'amplification, tandis que le moteur pas à pas effectue un mouvement droite/gauche par exemple un pas de pèlerin, afin ensuite de tourner dans sa direction prédominante lorsque le couple de rotation maximal est sous-dépassé.

7. Dispositif électrique d'entraînement selon la revendication 6, **caractérisé en ce que** le mouvement droite/gauche se limite à un nombre de degré prédéterminé.

8. Dispositif électrique d'entraînement selon l'une quelconque des revendications précédentes, **caractérisé en ce que** le dispositif d'entraînement est une pièce à main avec une pièce coudée pour la médecine dentaire, dans laquelle est logée une série d'arbres d'entraînement, qui peuvent être entraînés par le moteur pas à pas (1).

9. Procédé pour l'auto-étalonnage d'un dispositif électrique d'entraînement avec les caractéristiques selon l'une quelconque des revendications précédentes, **caractérisé par** des étapes de procédé suivantes :
a. alimenter le moteur (1) avec un courant minimal inférieur au courant de décollage spécifique au moteur,
b. augmenter progressivement le courant selon des commutations du champ tournant définies,
c. détecter la valeur de courant, à laquelle le moteur (1) démarre.

10. Procédé selon la revendication 9, **caractérisé en ce que** la valeur de courant détectée est enregistrée en tant que courant de fuite et additionnée en plus au courant maximal introduit.

11. Procédé selon l'une des revendications 9 et 10, **caractérisé en ce que** l'auto-étalonnage lors de la mise en service du moteur (1) s'effectue lors d'un changement de l'instrument d'entraînement et/ou après une période de temps prédéterminée ou pouvant être définie.

12. Procédé selon l'une quelconque des revendications précédentes, **caractérisé en ce que** le courant de fuite sous forme d'un code, déjà appliqué de préférence au dispositif d'entraînement, peut être détecté de manière électronique, ou être entré manuellement par le médecin, le courant de fuite étant déjà déterminé en usine.
